(19) Europäisches Patentamt
European Patent Office
Office européen des brevets

(11) Veröffentlichungsnummer: **0 103 178**
**B1**

(12) # EUROPÄISCHE PATENTSCHRIFT

(45) Veröffentlichungstag der Patentschrift: **09.03.88**

(51) Int. Cl.⁴: **C 08 B 37/00**, C 12 P 19/06

(21) Anmeldenummer: **83107960.3**

(22) Anmeldetag: **11.08.83**

(54) **Isolierung mikrobieller Polysaccharide aus ihren Fettamin-Addukten.**

(30) Priorität: **14.08.82 DE 3230301**

(43) Veröffentlichungstag der Anmeldung: **21.03.84 Patentblatt 84/12**

(45) Bekanntmachung des Hinweises auf die Patenterteilung: **09.03.88 Patentblatt 88/10**

(84) Benannte Vertragsstaaten: **AT BE CH DE FR GB IT LI LU NL SE**

(56) Entgegenhaltungen:
**GB - A - 1 167 747**
**US - A - 3 422 085**
**US - A - 3 928 316**

(73) Patentinhaber: **HOECHST AKTIENGESELLSCHAFT, Postfach 80 03 20, D-6230 Frankfurt am Main 80 (DE)**

(72) Erfinder: **Fischer, Edgar, Dr., Assmannshäuser Weg 8, D-6000 Frankfurt am Main 71 (DE)**
Erfinder: **Schlingmann, Merten, Dr., Schneidhainer Strasse 32a, D-6240 Königstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Isolierung von mikrobiellen Polysacchariden aus ihren Addukten mit Fettaminen.

Durch Fermentation hergestellte extrazelluläre mikrobielle Polysaccharide haben aufgrund ihrer hervorragenden Eigenschaften industrielle Anwendung als Verdicker, Gelier- oder Suspendiermittel, Schutzkolloide oder wasserbindende Mittel gefunden. Bedingt durch das Herstellungsverfahren sind diese Produkte recht teuer, wobei die bisher bekannten aufwendigen Isolierverfahren erheblich zu deren hohem Preis beitragen.

Aus der US-PS 3 928 316 ist es bekannt, das anionische Heteropolysaccharid, das durch Fermentation mit Hilfe des Bakteriums Xanthomonas campestris NRRL B-1459 erhalten wurde, als wasserunlösliches Salz eines primären langkettigen Amins aus den angesäuerten verdünnten Fermentationslösungen zu isolieren. Sofern eine Spaltung dieses Salzes beabsichtigt ist, erfolgt sie mit alkoholischer Kalilauge, wobei das Kaliumsalz erhalten wird, das aber noch aminhaltig ist.

Die EP-A-103 177 betrifft ein Verfahren zur Abtrennung von mikrobiellen Polysacchariden aus ihren wässrigen Lösungen durch Fällung im sauren Medium als Addukt mit einem langkettigen Alkylamin, das dadurch gekennzeichnet ist, dass ein Amin der Formel

$$NR^1R^2R^3$$

eingesetzt wird, in der $R^1$ Alkyl mit 10–20 Kohlenstoffatomen und $R^2$ und $R^3$, die gleich oder verschieden sind, Methyl oder Ethyl bedeuten.

Die bekannten Methoden zur Aufspaltung der Amin-Addukte verlaufen zum Teil recht unvollständig und führen zur salzhaltigen Polymeren. Somit werden weitere Reinigungsoperationen erforderlich, die nicht zuletzt wegen der Aufarbeitung der Abwässer aufwendig sind. Die Behandlung der Addukte mit starken Alkalien kann auch zu Schädigungen des Polymers führen.

Die EP-A-103 176 betrifft ein Verfahren zur Isolierung von mikrobiellen Polysacchariden aus ihren Amin-Addukten durch Behandeln mit alkalischen Mitteln in Gegenwart einwertiger Alkanole mit 1 bis 3 Kohlenstoffatomen, das dadurch gekennzeichnet ist, dass als alkalisches Mittel Ammoniak oder ein leicht flüchtiges Amin eingesetzt wird.

Die Erfindung betrifft demgegenüber ein Verfahren zur Isolierung von mikrobiellen Polysacchariden aus ihren Fettamin-Addukten, das dadurch gekennzeichnet ist, dass diese Addukte in Gegenwart eines Alkohols mit einem in diesem Alkohol löslichen oder thermisch leicht dissoziierenden Salz von Ammoniak oder einem leicht flüchtigen Amin, wobei das Amin einen Siedepunkt unter 150 °C bei Normaldruck hat, behandelt werden.

Im folgenden werden bevorzugt Ausgestaltungen der Erfindung näher erläutert:

Als Ausgangsmaterialien sind solche Addukte bevorzugt, die sich von Xanthan als mikrobiellem Polysaccharid und einem primären oder tertiären Fettalkylamin (mit zwei kurzkettigen Alkylresten) ableiten. Vorteilhaft werden diese Addukte im feuchten Zustand, beispielsweise als Presskuchen, eingesetzt, wie sie bei der Isolierung der mikrobiellen Polysaccharide aus den Fermentationslösungen anfallen. Es entfällt so das Trocknen der Addukte, das nicht nur beachtliche Energiemengen erfordert, sondern auch das Löslichkeits- und Quellvermögen der Addukte beeinträchtigen kann. Die geringen Wassermengen, die durch den Einsatz der feuchten Addukte eingebracht werden, stören beim erfindungsgemässen Verfahren nicht.

Als Alkohole eignen sich grundsätzlich alle, die ein hinreichendes Lösevermögen für die genannten Salze haben oder in denen diese Salze thermisch leicht dissoziieren können. Bevorzugt sind Alkohole mit einem Siedepunkt bei Normaldruck bis etwa 100 °C, insbesondere niedere Alkanole, vor allem Methanol, Ethanol, n- und iso-Propanol. Als Alkohole werden vorteilhaft die handelsüblichen technischen Qualitäten eingesetzt, wobei auch hierfür gilt, dass die geringen Wassermengen, die diese Produkte enthalten, das erfindungsgemässe Verfahren nicht beeinträchtigen. Grössere Wassermengen sind jedoch nicht vorteilhaft, da die freien mikrobiellen Polysaccharide, insbesondere das Xanthan, in Wasser löslich, in Alkoholen jedoch unlöslich sind. Ein höherer Wassergehalt im Medium könnte somit die Aufarbeitung erschweren.

Unter leicht flüchtigen Aminen, deren Salze eingesetzt werden, werden solche mit einem Siedepunkt unter 150 °C bei Normaldruck verstanden. Salze von Aminen mit höherem Siedepunkt sind weniger zweckmässig. Bevorzugt sind deshalb Salze primärer Alkylamine mit einem Alkylrest bis zu 6 Kohlenstoffatomen, sekundärer Amine mit gleichen oder verschiedenen Alkylresten mit bis zu 4 Kohlenstoffatomen und tertiärer Amine mit gleichen oder verschiedenen Alkylresten mit bis zu 3 Kohlenstoffatomen. Besonders bevorzugt sind die Salze von Trimethylamin, Triethylamin, Dimethylethylamin und Diethylmethylamin.

Als Salze sind thermisch leicht dissoziierende Produkte wie Ammoniumcarbonat, Ammoniumformiat, Ammoniumacetat, Triethylammoniumacetat, Trimethylammoniumformiat und Triethylammoniumformiat bevorzugt. Für thermisch stabilere Salze kommt es darauf an, dass sie in dem Alkohol bei der gewählten Verfahrenstemperatur eine hinreichende Löslichkeit besitzen, was durch einfache Vorversuche leicht feststellbar ist. Brauchbar sind beispielsweise Ammoniumchlorid, -bromid oder -nitrat, ferner die analogen Mono-, Di- und Triethylammoniumsalze.

Wenn das Addukt eines primären oder sekundären Fettamins gespalten wird bzw. wenn sich das eingesetzte leicht dissoziierende Salz von Ammoniak oder einem primären oder sekundären Amin ableitet, ist darauf zu achten, dass die freigesetzten Amine mit reaktiven Gruppen, insbesondere Estergruppen, der mikrobiellen Polysaccharide reagieren können. In solchen Fällen arbeitet man

deshalb zweckmässig bei niederen bis mässig erhöhten Temperaturen.

Bei Verwendung von Salzen tertiärer Amine, auch leicht dissoziierenden, und beim Einsatz von Addukten tertiärer Amine kann hingegen bei erhöhter Temperatur gearbeitet werden, ohne dass es zu einer Produktschädigung kommt. Höhere Temperaturen sind bevorzugt, da sie die Verfahrensdauer erheblich verkürzen.

Die Reaktion wird zweckmässig bei der Rückflusstemperatur des Reaktionsgemischs durchgeführt. Insbesondere beim Einsatz niederer Alkohole kann unter Anwendung von Druck gearbeitet werden.

Zur Spaltung der Addukte wird mindestens die äquimolare Menge, bezogen auf den Amingehalt im Addukt, an Salz eingesetzt, vorteilhaft ein mehr oder weniger grosser Überschuss, beispielsweise die zwei- bis zehnfache molare Menge. Da die freigesetzten mikrobiellen Polysaccharide in der alkoholischen Salzlösung nicht löslich sind, stört ein Salzüberschuss nicht, solange er gelöst bleibt, da dann die Lösung wieder in das Verfahren zurückgeführt werden kann. Diese Verfahrensweise kommt auch für eine kontinuierliche Ausgestaltung in Betracht.

Die mikrobiellen Polysaccharide fallen in einer leicht abtrennbaren Form an und liegen nach dem Trocknen weitgehend in der freien Säureform vor. Die Produkte zeichnen sich durch sehr günstige Viskositätseigenschaften aus.

Die den Alkohol und das abgespaltene Amin enthaltende flüssige Phase lässt sich destillativ aufarbeiten. Das aus dem Addukt abgespaltene Amin kann unmittelbar wieder zur Abtrennung mikrobieller Polysaccharide aus ihren Fermentationslösungen eingesetzt werden. Der Alkohol kann wieder in den Prozess zurückgeführt werden.

In den folgenden Beispielen beziehen sich Teile und Prozentangaben auf das Gewicht, sofern nichts anderes angegeben ist. Volumenteile stehen zu Gewichtsteilen im Verhältnis von 1 zu kg.

Zunächst wird die nicht-literaturbekannte Herstellung eines Addukts aus Xanthan und einem tertiären Fettalkylamin beschrieben (EP-A-103 177).

Als Produktionsstamm diente Xanthomonas campestris NRRL B-1459. Zur Vorkultur wurde eine Agarkultur in ein Glucose-Pepton-Medium überimpft und darin im Schüttelkolben bei 30 °C bebrütet. Diese Kultur diente als Inoculum (3%) für einen 10-Liter-Fermenter, dessen Nährmedium 3–5% Glucose oder Saccharose, 0,15–0,25% Cornsteep (Maisquellwasser), 0,1 bis 0,2% Natriumnitrat, 0,1% Dikaliumphosphat und 0,05% Magnesiumsulfat-Hydrat enthielt. Der beimpfte Fermenter wurde auf 28 °C gehalten und unter Rühren (400 U/min) mit 10 l Luft/min belüftet. Nach etwa 36 Stunden enthielt das Fermentationsmedium 18–20 g Xanthan pro Liter.

In 100 g einer Xanthanlösung mit einem Polysaccharidgehalt von 1,8% wurden 0,85 g Talgfettalkyl-dimethylamin (C-Kettenverteilung in Talgfettalkylrest: etwa 5% $C_{14}$; 30% $C_{16}$; 65% $C_{18}$) eingerührt. Nach Zugabe von 2,5 g 2-N-Essigsäure gerann die erhaltene Dispersion und das Addukt schied sich in Form von zunächst stark gequollenen Fladen ab, die aber beim Nachrühren rasch desolvatisierten. Das Addukt wurde abfiltriert, mit entionisiertem Wasser nachgewaschen und durch Abpressen entwässert. Es wurden 6,2 g eines feuchten Presskuchens erhalten, der 1,8 g Xanthan und 0,43 g Amin enthielt.

Beispiel 1

100 Teile eines feuchten Presskuchens, bestehend aus dem Addukt von Hexadecyldimethylamin an Xanthan, das 32% Xanthan enthält, wurden in einem mit einem Schneidepropeller ausgerüsteten Mixer zusammen mit 4000 Teilen Methanol zerkleinert und durch Zugabe von 100 Teilen Ammoniumbromid 6 Stunden unter Rückfluss erhitzt. Das dabei in faseriger Form freigesetzte Xanthan wurde abfiltriert und mit Methanol bis zur praktischen Bromidfreiheit gewaschen. Der Filterkuchen wurde getrocknet und zerkleinert. Die Ausbeute an Xanthan war praktisch quantitativ (31,9 Gewichtsteile). Das Produkt war in entionisiertem Wasser vollkommen löslich.

Beispiel 2

Es wurde wie in Beispiel 1 gearbeitet, jedoch ein feuchter Presskuchen eines Adduktes von Hexadecylamin und Xanthan und 3200 Teile Methanol sowie 100 Teile Ammoniumacetat eingesetzt. Das praktisch quantitativ erhaltene Xanthan löste sich vollständig in entionisiertem Wasser.

Die Beispiele 3–7 wurden analog Beispiel 1 mit den angegebenen Abänderungen ausgeführt. Es wurden jeweils 100 Teile Addukt eingesetzt. Das Produkt war immer in entionisiertem Wasser löslich.

| Beispiel | Xanthanaddukt mit | Alkohol (Teile) | Salz (Teile) |
|---|---|---|---|
| 3 | Cocosfettalkyldimethylamin | iso-Propanol (2500) | Trimethylammoniumacetat (170) |
| 4 | Talgfettalkylamin | Methanol (4000) | Ammoniumformiat (120) |
| 5 | Hexadecylamin | Methanol (3000) | Ammoniumhydrogencarbonat (80) |
| 6 | Sojafettalkyldimethylamin | Ethanol (3500) | Methyl-diethylammoniumacetat (180) |
| 7 | Talgfettalkyldimethylamin | Methanol (3500) | Triethylammoniumchlorid (200) |

**Patentansprüche**

1. Verfahren zur Isolierung von mikrobiellen Polysacchariden aus ihren Fettamin-Addukten, dadurch gekennzeichnet, dass diese Addukte in Gegenwart eines Alkohols mit einem in diesem

Alkohol löslichen oder thermisch leicht dissoziierenden Salz von Ammoniak oder einem leicht flüchtigen Amin, wobei das Amin einen Siedepunkt unter 150 °C bei Normaldruck hat, behandelt werden.

2. Verfahren nach Anspruch 1, dadurch gekennzeichnet, dass ein Alkohol mit einem Siedepunkt bei Normaldruck bis etwa 100 °C eingesetzt wird.

3. Verfahren nach Anspruch 1 und 2, dadurch gekennzeichnet, dass der Alkohol ein niederes Alkanol ist.

4. Verfahren nach einem oder der Ansprüche 1 bis 3, dadurch gekennzeichnet, dass die Reaktion bei der Rückflusstemperatur des Reaktionsgemischs durchgeführt wird.

5. Verfahren nach einem oder mehreren der Ansprüche 1 bis 4, dadurch gekennzeichnet, dass das mikrobielle Polysaccharid Xanthan ist.

6. Verfahren nach einem oder mehreren der Ansprüche 1 bis 5, dadurch gekennzeichnet, dass das Fettamin ein primäres Fettalkylamin ist.

7. Verfahren nach einem oder mehreren der Ansprüche 1 bis 5, dadurch gekennzeichnet, dass das Fettamin ein tertiäres Fettalkylamin mit zwei kurzkettigen Alkylresten ist.

**Claims**

1. A process for isolating microbial polysaccharides from their fatty amine adducts, which comprises treating these adducts in the presence of an alcohol with such a salt of ammonia or of a highly volatile amine as is soluble or thermally readily dissociated in this alcohol, the amine having a boiling point below 150 °C at atmospheric pressure.

2. The process as claimed in claim 1, wherein the alcohol used has a boiling point of up to about 100 °C at atmospheric pressure.

3. The process as claimed in claims 1 and 2, wherein the alcohol is a lower alkanol.

4. The process as claimed in any of claims 1 to 3, wherein the reaction is carried out at the reflux temperature of the reaction mixture.

5. The process as claimed in one or more of claims 1 to 4, wherein the microbial polysaccharide is xanthan.

6. The process as claimed in one or more of claims 1 to 5, wherein the fatty amine is a primary fatty alkylamine.

7. The process as claimed in one or more of claims 1 to 5, wherein the fatty amine is a tertiary fatty alkylamine having two short-chain alkyl radicals.

**Revendications**

1. Procédé d'isolement de polysaccharides microbiens à partir de leurs produits d'addition (adducts) d'amines grasses, caractérisé en ce qu'on traite ces produits d'addition en présence d'un alcool avec un sel d'ammoniac soluble dans cet alcool ou se dissociant facilement à la chaleur ou une amine très volatile, dans lequel l'amine présente un point d'ébullition inférieur à 150 °C à pression normale.

2. Procédé selon la revendication 1, caractérisé en ce qu'on utilise un alcool ayant un point d'ébullition à pression normale allant jusqu'à environ 100 °C.

3. Procédé selon les revendications 1 et 2, caractérisé en ce que l'alcool est un alcanol inférieur.

4. Procédé selon l'une des revendications 1 à 3, caractérisé en ce qu'on conduit la réaction à la température de reflux du mélange réactionnel.

5. Procédé selon une ou plusieurs des revendications 1 à 4, caractérisé en ce que le polysaccharide microbien est le xanthane.

6. Procédé selon une ou plusieurs des revendications 1 à 5, caractérisé en ce que l'amine grasse est une alcoylamine grasse primaire.

7. Procédé selon une ou plusieurs des revendications 1 à 5, caractérisé en ce que l'amine grasse est une alcoylamine grasse tertiaire avec deux radicaux alcoyle à chaîne courte.